# EUROPEAN PATENT APPLICATION

(11) **EP 4 520 383 A1**
(43) Date of publication of application: **12.03.2025**
(21) Application number: 23196258.0
(22) Date of filing: 08.09.2023
(51) Int. Cl.: A61M 37/00, A61K 8/02, A61Q 1/02, A61M 35/00

(54) **SEMI-PERMANENT TATTOO APPLICATOR FOR CURVED BODY PARTS**

(71) Applicant: inkbox ink Inc., Toronto, ON M5V 3G8 (CA)
(72) Inventor: KHALILI, Nazanin, Toronto, M5V 3G8 (CA); ADAM, Motaz, Toronto, M5V 3G8 (CA)
(74) Representative: Peterreins Schley

(57) **Abstract**

In a first aspect, the present disclosure relates to an applicator (100) for applying a pattern (130) comprising a semi-permanent colorant. The applicator comprises a carrier liner (110) and a pattern comprising a semi-permanent colorant. Further, the applicator comprises a first section (170), wherein the first section is in the shape of a trapezoid or an annular sector.

## Description

### Technical Field

The present invention relates to the field of tattoos. More specifically, the present invention relates to semi-permanent tattoos.

### Background

Temporary or semi-permanent tattoo or body inks have been used throughout human history to decorate the body. Generally, temporary or semi-permanent tattoos are transferred to the skin through the direct exposure of the skin to tattoo ink over a designated incubation period. The quality of the transferred image and its duration on the skin can depend on the ink distribution profile in the outer layer of the skin called the stratum corneum, or distribution profile in the epidermis. Current manufacturing processes for pre-fabricated tattoo designs include the utilization of topical formulations in many forms, including flexographic or gravure printing inks, stencils, and inkjet printers.

For example, semi-permanent tattoos may be applied using semi-permanent colorants, such as genipin and genipin derivatives. Genipin may chemically bind to the skin (mammalian, in particular human) resulting in a long-lasting blue coloration of the skin.

The semi-permanent colorant may be incorporated into an applicator, e.g. a sticker, to facilitate application of a semi-permanent tattoo by a user. The applicator may have a predefined pattern comprising the semi-permanent colorant, which defines the semi-permanent tattoo's design. For the manufacturing of an applicator for a semi-permanent tattoo, the semi-permanent colorants may be mixed with other compounds, such as solvents, to form a temporary tattoo ink. The temporary tattoo ink may then be printed in the form of the predefined pattern onto an adhesive layer provided on a carrier liner.

To apply the temporary tattoo ink to the skin, the applicator needs to be in contact with the skin for a significant amount of time. However, if the applicator moves out of place during the application process or the applicator is applied in imperfect skin alignment, the image is disfigured.

The present disclosure relates to improvements to semi-permanent tattoo applicators.

### Summary

In a first aspect, the present disclosure relates to an applicator for applying a pattern comprising a semi-permanent colorant. The applicator comprises a carrier liner and a pattern comprising a semi-permanent colorant. Further, the applicator comprises a first section, wherein the first section is in the shape of a trapezoid or an annular sector.

In some embodiments, the applicator comprises a backing layer comprising a fabric.

In some embodiments, the trapezoid or annular sector comprises at least one base comprising at least one notch.

In some embodiments, the backing layer comprises a non-woven fabric, more specifically a non-woven thermoplastic and in particular a non-woven polyester.

In some embodiments, the backing layer, in particular the fabric, is pliant.

In some embodiments, the backing layer is configured to be adhesive to itself.

In some embodiments, the backing layer is configured to be non-adhesive to skin.

In some embodiments, the backing layer has a thickness between about 5 mils to about 30 mils.

In some embodiments, the backing layer has a tensile strength between about 0.5 MPa to about 10 MPa, more specifically between about 1 MPa to about 7 MPa and in particular between about 2 MPa to about 5 MPa.

In some embodiments, the backing layer has an elongation at yield of at least 110%, more specifically at least 115% and in particular at least 120%.

In some embodiments, the backing layer has an elongation at yield of between about 110% to about 200%, more specifically between about 115% to about 180 % and in particular between about 120% to about 160%, more specifically along an axis within the backing layer's planar extension and in particular along two perpendicular axis within the backing layer's planar extension..

In some embodiments, the first section comprises at least two, and in particular two, notches in a first base and/or the first section comprises at least one notch, more specifically at least two notches and in particular two notches, in a second base.

In some embodiments, the notches comprise an apex, wherein a line between an apex of a first notch on the first base and a second notch on the second base is a radius of the annular sector or trapezoid.

In some embodiments, the first base has a first length between a first leg and a second leg of the trapezoid or annular sector, and wherein the distance between the first leg and the apex of the first notch is between about 0.2 times to about 0.4 times of the first length, more specifically wherein the first base comprises a first additional notch and wherein the distance between the second leg and the apex of the first additional notch is between about 0.2 times to about 0.4 times of the first length.

In some embodiments, the second base has a second length between the first leg and the second leg of the trapezoid or annular sector and wherein the distance between the first leg and the apex of the second notch is between about 0.2 times to about 0.4 times of the second length, more specifically wherein the second base comprises a second additional notch and wherein the distance between the second leg and the apex of the second additional notch is between about 0.2 times to about 0.4 times of the second length.

In some embodiments, the backing layer comprises a fabric layer and an adhesive layer, wherein the adhesive layer of the backing layer is attached to the carrier liner in the first section.

In some embodiments, the first section is connected to a second section comprising at least one strap, more specifically at least two straps and in particular three straps.

In some embodiments, the adhesive layer of the backing layer is attached to a first release liner in the second section.

In some embodiments, the second section, in particular the at least one strap, is attached to a leg of the trapezoid or of the annular sector.

In some embodiments, a length of a leg of the trapezoid or of the annular sector is between about 5 cm to about 50 cm, more specifically between about 10 cm to about 40 cm.

In some embodiments, the carrier liner is only present in the first section.

In some embodiments, the carrier liner comprises a carrier liner adhesive layer, wherein the pattern is provided on and/or in the carrier liner adhesive layer.

In some embodiments, the carrier liner adhesive layer is attached to a second release liner, in particular a different release liner than the first release liner of the second section.

In some embodiments, the carrier liner is non-porous.

In a second aspect, the present disclosure relates to a method for manufacturing an applicator for applying a pattern comprising a semi-permanent colorant. The method comprises applying a temporary tattoo ink on an adhesive layer provided on a carrier liner to provide a pattern. The carrier liner is in the shape of a trapezoid or an annular sector.

In some embodiments according to the second aspect, the method optionally comprises cutting the carrier liner into a trapezoid or annular sector; or providing the carrier liner as a trapezoid or annular sector.

In some embodiments, the method comprises providing a backing layer comprising an adhesive layer and laminating the carrier liner onto the adhesive layer of the backing layer.

In some embodiments according to the second aspect, the cutting step follows the application of the temporary tattoo ink on the adhesive layer and in particular may follow the lamination of the carrier liner with its release liner.

In some embodiments, the method comprises kiss-cutting a release liner of the backing layer in the shape of a trapezoid or annular sector and removing the kiss-cut release liner.

In some embodiments according to the second aspect, the method comprises cutting the backing layer to form a first section and a second section, wherein the first section has a trapezoidal or annular sector shape and the second section comprises at least one strap, wherein the first section is connected to the second section and the connection between the first section and the section is kiss-cut.

In some embodiments according to the second aspect, cutting the backing layer may include forming a plurality of straps in the second section.

In some embodiments according to the second aspect, the method includes laminating the adhesive layer onto the backing layer, in particular wherein the adhesive layer of the backing layer is a double-sided transfer tape.

In some embodiments, the method comprises attaching a release liner to the adhesive layer, in particular by lamination, such that the release liner has a surface contacting the adhesive layer and the pattern.

In a third aspect the present disclosure relates to a use of a backing layer for preventing or reducing wrinkling and/or buckling of an applicator comprising a pattern comprising a semi-permanent colorant during application of the applicator onto skin.

### Brief Description of the Figures

**Fig. 1** shows a frontal-view drawing of an applicator according to the first aspect.
**Fig. 2** shows a transversal cut of a first section of an applicator according to the first aspect.
**Fig. 3** shows a transversal cut of a second section of an applicator according to the first aspect.

### Detailed Description

Hereinafter, a detailed description will be given of the present disclosure. The terms or words used in the description and the aspects of the present disclosure are not to be construed limitedly as only having common-language or dictionary meanings and should, unless specifically defined otherwise in the following description, be interpreted as having their ordinary technical meaning as established in the relevant technical field. The detailed description will refer to specific embodiments to better illustrate the present disclosure, however, it should be understood that the presented disclosure is not limited to these specific embodiments.

Semi-permanent colorants may be incorporated into applicators, e.g. stickers, to facilitate application of a semi-permanent tattoo by a user. The applicator may have a predefined pattern comprising the semi-permanent colorant, which defines the semi-permanent tattoo's design. For the manufacturing of an applicator for a semi-permanent tattoo, the semi-permanent colorants may be mixed with other compounds, such as solvents, to form a temporary tattoo ink. The temporary tattoo ink may be printed in the form of the predefined pattern onto an adhesive layer provided on a carrier liner. Subsequently, the adhesive layer may then be placed on the part of the skin intended to display a semi-permanent tattoo, where the semi-permanent colorant may diffuse into the skin.

The application of semi-permanent tattoos using the aforementioned applicators offers multiple advantages. Firstly, it allows the user to apply a semi-permanent tattoo in a single step, without considerable effort. Additionally, applying the temporary tattoo ink to the adhesive layer by printing, notably inkjet printing, allows for high-throughput manufacturing with precise designs.

However, if the applicator moves out of place during the application process or if the applicator is applied in imperfect skin alignment, the image may be distorted. Small applicators which are commonly applied to "flat" body parts commonly exhibit good skin alignment and a relatively low risk of moving out of place during the application duration. However, larger designs(and thus larger applicators) frequently encounter the problem that, due to their size, have to be applied to curved body parts. These larger designs may not properly align to the skin and parts thereof may move during the application process. For example, the lower arm is a highly sought after body part for tattoo application, but represents a highly curved surface, or in other words a body part exhibiting a variable circumference, to which a conventional larger sized temporary tattoo patch may not align. As a result, there is a need for an applicator suitable for application to curved body parts, in particular large, curved body parts.

Accordingly, in a first aspect, the present disclosure relates to an applicator for applying a pattern comprising a semi-permanent colorant. The applicator comprises a carrier liner and a pattern comprising a semi-permanent colorant. Further, the applicator comprises a first section, wherein the first section is in the shape of a trapezoid or an annular sector.

The term "trapezoid" is well-known and i.a. (inter alia) refers to its common meaning in the art. Additionally or alternatively, the term trapezoid refers to a geometric shape comprising four corners, wherein a first line connecting a first corner and a second corner is parallel to a second line connecting a third corner and fourth corner, and wherein a first angle formed at the first point by the first line and a third line connecting the first corner and the third corner is greater than 90° and wherein a second angle formed at the second corner by the first line and a fourth line connecting the second corner and the fourth corner is also greater than 90°, in particular the first angle and the second angle may be the same angle, e.g. the trapezoid may be an isosceles trapezoid. The first corner and second corner may be connected by a first base and the third corner and the fourth corner may be connected by a second base. The first corner and the third corner may be connected by a first leg and the second corner and the fourth corner may be connected by a second leg. In some embodiments, the first base and/or the second base are curved, in particular the first base and the second base are convex. In some embodiments, the first leg and the second leg may be straight. The term "base" as used herein, also refers to the circular arcs of the annular sector. The term "annular sector" is well-known and i.a. attributed its common meaning in the art. Additionally or alternatively, the term "annular sector" relates to a section of an annulus bordered by two lines, wherein each line is a radius of the annulus.

The trapezoid or annular sector shape may improve the alignment of the applicator to curved body parts. In particular, such shapes may allow wrapping the applicator around the lower arm (forearm) to apply a quarter- or half-sleeve semi-permanent tattoo. The lower arm may be approximated as a truncated conus, which has a broader diameter or circumference at the elbow compared to the wrist. When wrapping a rectangular applicator into a shape other than a cylinder, e.g. around a lower arm, the applicator material tends to buckle and wrinkle due to internal shear forces in the applicator. On the other hand, the trapezoidal or annular sector shape allows wrapping of the applicator around a conical shape, such as a lower arm, with reduced buckling or wrinkling of the applicator. The term "wrinkling" is well-known and i.a. attributed its common meaning in the art. Additionally or alternatively, the term wrinkling relates to a structure deforming by forming small lines or folds. The term "buckling" is well-known and i.a. attributed its common meaning in the art. Additionally or alternatively, the term buckling relates to a structure deforming by bending, wherein the bend cannot be aligned with the user's skin in at least some areas, in particular under formation of wrinkles.

Semi-permanent tattoos are typically self-applied by the user. As a result, the application process occurs in a relatively uncontrolled environment. While typical smaller and rectangular applicators may be easy to apply, an applicator for curved body parts, such as the lower arm or lower leg, may require significantly longer and more steps to be completely applied. Known applicators may consist of relatively small material layers and may therefore be prone to wrinkling during a multi-step application process. However, typical material layers that can be added for stability, such as paper or cardboard may increase the risk of buckling as they have low flexibility in their planar extension. It has been found that fabrics may provide mechanical stability against wrinkling, while not leading to an increase of buckling. Accordingly, in some embodiments, the applicator comprises a backing layer comprising a fabric.

In some embodiments, the backing layer comprises a non-woven fabric, more specifically a non-woven thermoplastic, even more specifically a non-woven synthetic thermoplastic, and in particular a non-woven polyester.

In some embodiments, the backing layer, in particular the fabric, is pliant. As described above the backing layer may be elastic in its planar extension. In some embodiments, the backing layer has an elongation at yield of at least 110%, more specifically at least 115% and in particular at least 120%, more specifically along an axis within the backing layer's planar extension and in particular along two perpendicular axis within the backing layer's planar extension.

In some embodiments, the backing layer has an elongation at yield of between about 110% to about 200%, more specifically between about 115% to about 180 % and in particular between about 120% to about 160%, more specifically along an axis within the backing layer's planar extension and in particular along two perpendicular axis within the backing layer's planar extension..

In some embodiments, the backing layer is configured to be adhesive to itself. In some embodiments, the backing layer is configured to be non-adhesive to skin. For example, cohesive bandages, such as 3M ^{™} Coban ^{™} by the Company 3M Corp., Saint Paul, Minnesota, USA, is a self-adherent wrap, while not adhering to skin. The self-adhering property, while being non-adhesive to skin may facilitate the application process. In particular, the user may be able to handle the applicator without the backing layer attaching to the user, however, the user may secure the backing layer to itself when finishing the application process.

In some embodiments, the backing layer has a thickness between about 5 mils to about 30 mils.

In some embodiments, the backing layer has a tensile strength between about 0.5 MPa to about 10 MPa, more specifically between about 1 MPa to about 7 MPa and in particular between about 2 MPa to about 5 MPa.

Additionally, the applicator shape may be adapted to reduce the risk of buckling and/or wrinkling. Incisions could be applied to the applicator to reduce the shear stress therein. However, these incisions are visible in the final semi-permanent tattoo or can lead to overlaps of the applicator during application which would lead to distortion of the final semi-permanent tattoo. However, it has been found that by including notches in the applicator, the shear stress may be partially relieved, while minimally affecting the final semi-permanent tattoo. Accordingly, in some embodiments, the trapezoid or annular sector comprise at least one base comprising at least one notch. In some embodiments, the first section comprises at least two and in particular two notches in the first base. Additionally or alternatively the first section may comprise at least one notch, more specifically at least two notches and in particular two notches in the second base.

In some embodiments, the notches comprise an apex, wherein a line between an apex of a first notch on the first base and a second notch on the second base is a radius of the annular sector or the trapezoid. The term "radius of the trapezoid" herein relates to a line that has the same origin as the first leg and second leg. The term "radius of the trapezoid" relates to a line that intersects with the first leg and the second leg in the same point where the first leg and second leg intersect, (if the first leg and second leg were extended as infinite lines). In some embodiments, at least one of the apexes is the angle of intersection of two circular arcs. In particular, wherein each notch comprises two edges, wherein each edge is defined by a circular arc and wherein the angle of intersection of the circular arcs is the apex. The term circular arc is well-known and i.a. relates to its common meaning in the art. Additionally or alternatively, the term "circular arc" relates to a section of a circle. In some embodiments, the angle of intersection at the apex is between about 10 ° to about 90°, more specifically about 20 ° to about 70 ° and in particular between about 30 ° to about 50°. The aforementioned alignment of the notches and angle of intersections at the apex may be particularly effective in reducing wrinkling and/or buckling. In some embodiments, the applicator may comprise at least one notch and a backing layer. The backing layer in combination with the at least one notch may comprise a fabric but may also be of a different material.

In some embodiments, the first base has a first length between the first leg and the second leg of the trapezoid or annular sector, and wherein the distance between the first leg and the apex of the first notch is between about 0.2 times to about 0.4 times of the first length, more specifically wherein the first base further comprises a first additional notch and wherein the distance between the second leg and the apex of the first additional notch is between about 0.2 times to about 0.4 times of the first length. In some embodiments, the second base has a second length between the first leg and the second leg of the trapezoid or annular sector, and wherein the distance between the first leg and the apex of the second notch is between about 0.2 times to about 0.4 times of the second length, more specifically wherein the second base further comprises a second additional notch and wherein the distance between the second leg and the apex of the second additional notch is between about 0.2 times to about 0.4 times of the second length. In some embodiments, the distance between the first notch and the first leg is the same as between the first additional notch and the second leg and/or the distance between the second notch and the first leg is the same as between the second additional notch and the second leg.

The aforementioned placement of the notches may be particularly effective in reducing wrinkling and/or buckling. Furthermore, they may provide the user with guidance for placing the applicator. For example, the applicator may be used to apply a semi-permanent tattoo to the lower arm or to the lower leg. The user may position their wrist between the first notch and the first additional notch and their upper arm between the second notch and the second additional notch, hence aligning the applicator and the resulting semi-permanent tattoo on their arm. The notches may therefore also act as positional markers.

The at least one notch may be in particular combined with the fabric for an improved application process and result. Both the backing layer comprising a fabric and the at least one notch may reduce the risk of wrinkling. In particular, the fabric may provide sufficient strength to the applicator and the notches may reduce shear stress in the applicator.

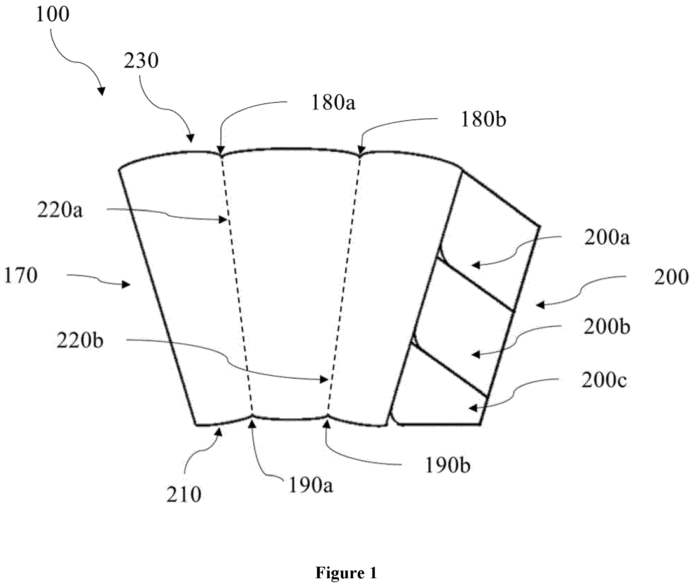

A frontal-view depiction of an applicator according to the first aspect is shown in Fig. 1. The applicator 100 of Fig. 1 comprises a first section 170. The first section comprises four notches: a first notch 190a, a first additional notch 190b, a second notch 180a, a second additional notch 180b. The first section has a trapezoidal shape with a convexly curved second base 230 and an approximately straight first base 210. The first base 210 and second base 230 are segmented into three curved sections, due to the presence of the notches. The notches 180a and 190a align along a radius 220a of the trapezoid and the notches 180b and 190b align along another radius 220b of the trapezoid, as marked by the line 220a and 220b respectively. The first section 170 is attached to a second section 200 formed by three straps 200a, 200b and 200c.

In some embodiments, the carrier liner is non-porous. The semi-permanent colorant from the pattern may require not only time but also humidity to effectively diffuse from the applicator to/into the skin. Accordingly, it may be desirable that the user's sweat is retained between the applicator and the user's skin. However, a non-porous carrier liner may be even more prone to buckling and wrinkling and therefore require additional measures to improve the application process, e.g. notches and/or fabric.

In some embodiments, the first section is connected to a second section comprising at least one strap, more specifically at least two straps and in particular three straps. Such straps 200a, 200b and 200c are illustrated in Fig. 1. The straps may be used to fasten the applicator to the curved body part and thereby securing the applicator from becoming displaced. The straps may be for example attached to the first section at the end of the wrapping process. The straps may exhibit a higher adhesion strength to the first section, than the first section has to the user's skin. In other words, the adhesion between the first section and the user's skin may be insufficient to ensure that the applicator does not detach from the skin. However, by attaching the straps to the first section, the applicator may be additionally attached to the skin by a compressive strength applied by the wrapping, thereby providing an improved attachment of the applicator to the user's skin. In some embodiments, the second section, in particular at least one strap, is attached to a leg of the trapezoid or a radius of the annular sector. The term "leg" as used herein also refers to a radius of the annular sector. Further, the straps may secure the first leg of the first section to the skin, which may otherwise become loose leading to a compromised semi-permanent tattoo. Accordingly, in some embodiments, the at least one strap may cover at least 70%, more specifically at least 80% and in particular at least 90% of the length of the second leg of the first section. Having multiple straps as opposed to a large single strap may decrease the risk of wrinkling or buckling.

In some embodiments, the backing layer comprises a fabric layer and an adhesive layer, wherein the adhesive layer of the backing layer is attached to the carrier liner in the first section. In some embodiments, the adhesive layer of the backing layer is a double sided transfer tape, e.g. an adhesive film attached to a release liner on both sides.

In some embodiments, the adhesive layer of the backing layer is attached to a first release liner in the second section. Accordingly, the adhesive layer of the backing layer may attach the backing layer to the carrier liner in the first section. The adhesive layer of the backing layer of the second section may be used to secure the second section, in particular the at least one strap, to the first section during the application process. The first release liner may prevent the adhesive layer of the backing layer from becoming contaminated which may impair its adhesive properties. The first release liner may comprise a polymer or a paper.

In some embodiments, the backing layer may be continuous or integrally formed.

In some embodiments, a length of a leg of the trapezoid or the annular sector is between about 5 cm to about 50 cm, more specifically between about 10 cm to about 40 cm.

In some embodiments, the carrier liner comprises a carrier liner adhesive layer, wherein the pattern is provided on and/or in the carrier liner adhesive layer.

In some embodiments, the carrier liner is only present in the first section. The carrier liner may be required to provide the carrier liner adhesive layer to support the pattern and to retain the user's sweat between the applicator and the user's skin. It may therefore not be required in the second section and the second section, e.g. the at least one strap, may be more flexible without the carrier liner present.

In some embodiments, the carrier liner adhesive layer is attached to a second release liner, in particular a different release liner as the release liner of the second section. The second release liner of the carrier liner adhesive layer may protect the pattern disposed between it and the carrier liner. However, for an optimal result, the second release liner attached to the pattern may require a dedicated configuration to prevent patchiness of the semi-permanent tattoo as outlined below.

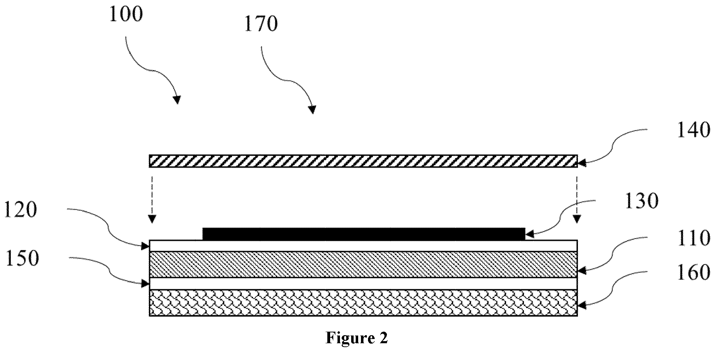

An exemplary exploded-view depiction of a transversal cut of a first section 170 of an applicator 100 according to the first aspect is shown in Fig.2. The first section 170 of the applicator 100 of Fig. 2 comprises a carrier liner adhesive layer 120 provided on a carrier liner 110. A pattern 130 is provided on the carrier liner adhesive layer 120. The second release liner 140 is provided on the pattern 130 and the adhesive layer 120 (depicted with a distance for overview purposes). A backing layer 160 is attached to the carrier liner by an adhesive layer of the backing layer 150.

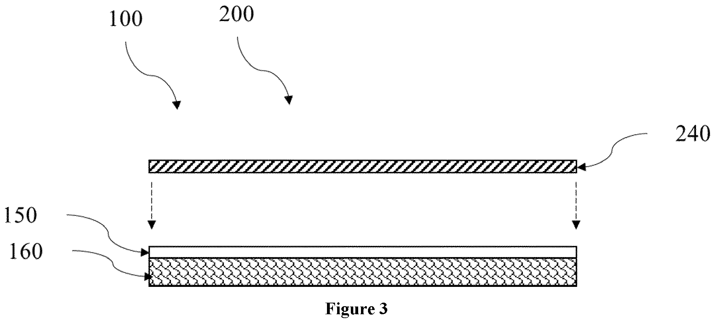

An exemplary exploded-view depiction of a transversal cut of a second section 200 of an applicator 100 according to the first aspect is shown in Fig. 3. The second section 200 of the applicator 100 of Fig. 3 comprises a backing layer 160 with an adhesive layer of the backing layer 150. The first release liner 240 is provided on the adhesive layer of the backing layer 150.

Accordingly, in some embodiments, the first section and the second section of the applicator comprise the backing layer. In some embodiments, the carrier liner, carrier liner adhesive layer and/or pattern are only present in the first section. In some embodiments, the applicator comprises the layers in the first section from bottom to top in the sequence: backing layer, adhesive layer of the backing layer, carrier liner, carrier liner adhesive layer, pattern and optionally second release liner. In some embodiments, the applicator comprises the layers in the seconds section from bottom to top in the sequence: backing layer, adhesive layer of the backing layer and optionally first release liner. Additional layers may be disposed between the layers, in particular between the backing layer and adhesive layer of the backing layer and/or between the carrier liner and carrier liner adhesive layer, however, in particular, no additional layers are disposed therebetween.

In some embodiments, the one or more semi-permanent colorant may comprise a colorant precursor. In some embodiments, the colorant precursor may comprise genipin or a genipin derivative. Accordingly, in some embodiments, the semi-permanent colorant may comprise genipin or a genipin derivative, in particular purified genipin. The term "purified genipin" may relate to a semi-permanent colorant comprising at least about 90 wt.-%, more specifically at least about 95 wt.-% and in particular at least about 98.5 wt.-% genipin, relative to the total weight of the semi-permanent colorant.

The term "colorant" is well-known and i.a. attributed its common meaning in the art. Additionally or alternatively, the term "colorant" may refer to a substance that changes the color of reflected or transmitted light as the result of wavelength-selective absorption. As used herein, "color" refers to wavelengths of electromagnetic radiation visible to the human eye. The term "semi-permanent colorant" is well-known and i.a. attributed its common meaning in the art. Additionally or alternatively, the term "semi-permanent colorant" may refer to a colorant that penetrates one or more layers of the skin and is unable to be removed from the skin without physical disruption or natural desquamation of the skin. For example, a semi-permanent colorant may penetrate the stratum corneum and/or epidermis and react with other molecules, e.g., other colorant molecules and/or other molecules present in the stratum corneum and/or epidermis, such that the semi-permanent colorant is immobilized within the stratum corneum and/or epidermis. For example, the semi-permanent colorant may cross-link with collagen found in the stratum corneum and/or epidermis. In some embodiments, the semi-permanent colorant may react with other molecules, e.g., other colorant molecules and/or other molecules present in the stratum corneum and/or epidermis, to form a molecule that is too large to diffuse out of the stratum corneum and/or epidermis. In some embodiments, wherein the semi-permanent colorant penetrates the stratum corneum and/or epidermis, the colorant residence time is determined by the natural skin desquamation process. In some embodiments, the semi-permanent colorant cannot be washed off, for example, by water, soap, and/or isopropanol. In some embodiments, a semi-permanent colorant includes a natural or synthetic pigment or dye. In some embodiments, a semi-permanent colorant can include a colorant precursor, for example, a molecule, such as genipin, that expresses color upon reaction with one or more other molecules.

As used herein, "genipin" refers to genipin extract, partially purified genipin extract, and/or purified genipin, as described further herein. Genipin is derived from an iridoid glycoside called geniposide, which is present in nearly 40 plant species. Genipin may be derived for example from the plant Genipa americana, in particular its fruits. The plant genipa americana is also known as jagua, caruto, huito, jenipapo, genipapa, guaitil, tapaculo, yigualti, shagua, xagua, maluco, vito, ñandipá or bi. Genipin may also be derived for example from Gardenia Jasminoides, in particular its fruits. Genipa is also sold under the names Jagua-extract or Genipa-food extract. Additionally, genipin is a precursor to gardenia blue that undergoes a free radical polymerization and/or oligomerization reaction upon exposure to amine groups to form a blue colorant (see, for example, Touyama et al., Chem. Pharm. Bull. 42:668-673, 1994; and Touyama et al., Chem. Pharm. Bull. 42(8): 1571-1578, 1994; both of which are incorporated by reference herein in their entireties). When placed in contact with skin, genipin can react with the amines in skin to generate the polymer-based and/or oligomer-based blue color in situ. The penetration profile of genipin can dictate the color concentration profile in the stratum corneum and/or epidermis as the polymer-based and/or oligomer-based blue colorant is too large to penetrate the skin at relevant concentrations.

As will be explained in more detail below, the applicators of the present disclosure may be manufactured by inkjet printing. In some embodiments, the second release liner of the first section comprises or is a film. A film may be in particular suitable for applicators manufactured by inkjet printing, compared to for example applicators manufactured by screen printing. Patterns applied by inkjet printing may be more delicate as they are applied in the form of tiny droplets, which dry to form spots. In some embodiments, the pattern may comprise a plurality of spots, wherein the plurality of spots may have an average maximum diameter between about 10 µm to about 500 µm, more specifically between about 50 µm to about 250 µm and in particular between about 75 µm to about 130 µm. The average maximum diameter of the spots may be determined using a microscope and averaged over 30 spots.

The film (the second release liner) may be a thin sheet of continuous material. The film may be in particular a non-fibrous film, as fibers, such as cellulose fibers, may increase the surface roughness of the film, which may in turn result in patchiness of the pattern. Similarly, the film may be non-porous, as pores may also increase the surface roughness of the film.

In some embodiments, the average surface roughness of the film may be less than 3 µm, more specifically less than 1 µm and in particular less than 500 nm, measured according to ISO 21920-2:2021. In some embodiments, the average surface roughness of the film may be between about 0.5 nm to about 500 nm, more specifically between about 1 nm to about 300 nm and in particular between about 3 nm to about 100 nm, measured according to ISO 21920-2:2021.

In some embodiments, the film (the second release liner) may have a thickness between about 10 µm to about 400 µm, more specifically between about 80 µm to about 250 µm and in particular between about 120 µm to about 180 µm. The aforementioned film thicknesses may provide a good balance between protection and flexibility. As will be explained in more detail below, the applicators of the present disclosure may be manufactured by inkjet printing. For the inkjet printing process and subsequent drying process, the carrier liner may be provided with a support layer attached to the carrier liner, which increase the stiffness of the carrier liner. The increased stiffness may improve the processability of the carrier liner, e.g. by preventing wrinkling of the carrier liner. However, subsequently, the support layer needs to be removed from the applicator, as otherwise the applicator may not properly align to the user's skin due to the increased stiffness. However, during removal, e.g. by delamination, the applicator may wrinkle. A film of increased thickness, which is laminated onto the applicator prior to the delamination, may aid in preventing wrinkling of the applicator, e.g. by increasing the applicator's stiffness. The same may apply when laminating the carrier liner onto the backing layer. Further, the carrier liner with the carrier liner adhesive layer may be provided in the form of large sheets which are printed at once and subsequently cut into the individual applicators. A film of greater thickness may also aid in allowing precise cutting without damage to the applicators. Further, the applicators may unintentionally curl after cutting due to absorption of water. The curling may be reduced using a thicker film.

In some embodiments, the second release liner may be provided on and/or attached to the pattern. In some embodiments, the second release liner may be provided on and/or be attached to the pattern and the carrier liner adhesive layer, in particular on the opposite side of the carrier liner adhesive layer whereto the carrier liner is attached. In some embodiments, the second release liner may comprise a pull-tab to facilitate the detachment of the second release liner. In some embodiments, the carrier liner may have a thickness between about 10 µm to about 300 µm, more specifically between about 30 µm to about 150 µm and in particular between about 40 µm to about 100 µm.

In some embodiments, the carrier liner may have a first face and a second face, wherein the carrier liner adhesive layer may be provided on at least about 50%, more specifically at least 70% and in particular at least 90% of the second face, relative to the total surface of the second face. In particular, the carrier liner adhesive layer may be provided on all of the at least one face of the carrier liner. The carrier liner adhesive layer covering a high proportion of the surface of the second face of the carrier liner may improve the adhesion of the carrier to the skin when applied thereto.

In some embodiments, the pattern may be provided on and/or in less than 95%, more specifically less than 90% and in particular less than 85% of a face of the carrier liner adhesive layer facing away from the carrier liner, relative to the total surface of the face of the carrier liner adhesive layer facing away from the carrier liner. In other words, the carrier liner adhesive layer may have a first face and a second face, and the first face of the adhesive may be in contact with the second face of the carrier liner and the pattern may be provided on the second face of the adhesive. Hence, at least part of the carrier liner adhesive layer is available to be brought into contact with the skin. Further, the carrier liner adhesive layer may not include the pattern itself, instead the pattern may be provided as a distinct entity. A carrier liner adhesive layer including the pattern may allow less semi-permanent colorant to diffuse into the skin compared to a pattern provided on a carrier liner adhesive layer. Yet, the pattern may have partially diffused into the carrier liner adhesive layer.

In some embodiments, the second release liner may be provided on about 70%, more specifically at least 90% and in particular fully covers the pattern and the face of the carrier liner adhesive layer facing away from the carrier liner, relative to the total area of the pattern and the face of the carrier liner adhesive layer facing away from the carrier liner. The second release liner may protect the pattern prior to application.

In some embodiments, the second release liner may comprise a non-adhesive material. In some embodiments, the film of the second release liner comprises, essentially consists or consists of a polymer, in particular polyethylene terephthalate. Polymer films, in particular polyethylene terephthalate films, may exhibit low surface roughness. Additionally, polymer films, in particular polyethylene terephthalate films, may be flexible yet durable, in particular tear resistant. Further, the applicators may unintentionally curl after cutting due to absorption of water. The curling may be reduced using a polymer film compared to a paper release liner, as it may absorb less water. In some embodiments, the second release liner may be flexible. In some embodiments, the second release liner may exhibit a Young's modulus between about 0.1 GPa to about 60 Gpa, more specifically 0.5 Gpa to about 10 Gpa and in particular, between about 1 Gpa to about 5 Gpa.

In some embodiments, the carrier liner comprises, essentially consists or consists of a polymer, more specifically a flexible polymer, in particular polyethylene. In some embodiments, the carrier liner may exhibit a Young's modulus between about 0.1 Gpa to about 60 Gpa, more specifically 0.5 Gpa to about 10 Gpa and in particular, between about 1 Gpa to about 5 Gpa.

The applicator may rely solely on residual solvents in the pattern and sweat to transfer the semi-permanent colorant from the pattern to the skin. In particular, the applicator may not require water to transfer the pattern, or semi-permanent colorant comprised within the pattern, to the skin. In some embodiments, the carrier liner comprises, essentially consists or consists of a water-resistant polymer or a waterproof polymer, in particular a waterproof polymer. Accordingly, in some embodiments, the carrier liner may be water-resistant or waterproof. The term "water-resistant carrier liner" within this disclosure refers to a carrier liner, that when in contact with a moist sponge cloth on a first face, does not show a detectable increase moisture on the second face after at least 10 minutes of contact. The term "waterproof carrier liner" within this disclosure refers to a carrier liner, that when in contact with a moist sponge cloth on a first face, does not show detectable increase in moisture on the second face after at least 1 hour of contact. The moist sponge cloth may be for example a sponge cloth comprising 75 cellulose and 25 % cotton, with a thickness in the dry state between about 2 mm to about 10 mm. The sponge cloth should be fully immersed in water for 5 minutes, and subsequently allowed to release excess water by being hung-up for 5 minutes, e.g. attached to a peg. For example, a "vileda Original Schwammtuch" by the company Vileda GmbH, Weinheim, Germany, may be used for the test. The moisture may be detected using a moisture meter, e.g. a MM7 Pin-Type Moisture Meter purchasable from the company General Tool Inc, Irvine California. An increase of moisture may be considered a "detectable increase in moisture" if the moisture increases by at least 15%, more specifically at least 20%. The temperature of the moist sponge cloth needs to be at least ambient temperature, to prevent condensation of water on the second face of the carrier liner.

In some embodiments, the carrier liner adhesive layer may comprise a rubber adhesive, a silicone adhesive, polyurethane adhesive, a hydrogel adhesive, a hydrocolloid adhesive, an acrylic adhesive and/or a dry adhesive. In particular, in some embodiments, the carrier liner adhesive layer comprises, essentially consists or consists of a rubber adhesive. For example, the carrier liner adhesive layer may comprise a styrene-copolymer adhesive. In some embodiments, the carrier liner adhesive layer may comprise a pressure-sensitive adhesive. A pressure-sensitive adhesive may be advantageous as it does not require activation, for example by water. The term "activation of an adhesive layer" within this disclosure refers to applying an external stimulus, except pressure, to the carrier liner adhesive layer, for the carrier liner adhesive layer to adhere to a surface, e.g. skin. The external stimulus may be one or more of heat, drying, shear, UV-light or the addition of other substances. In some embodiments, the carrier liner adhesive layer and/or pattern may be in direct contact with the second release liner, more specifically the film and/or a coating provided or comprised in the film.

Further, the adhesive may be configured not to transfer to the skin. For example, decals may comprise an adhesive layer provided on a carrier liner and an additional water-absorbent layer. A water-soluble slip layer may be arranged between the water-absorbent layer, e.g. porous paper, and the carrier liner. The water-soluble slip layer may allow the carrier liner adhesive layer and carrier to transfer from the water-absorbent layer to the skin upon application. However, the combination of the carrier liner with the carrier liner adhesive layer may leave a sticky residue on the skin. In some embodiments, the carrier liner adhesive layer may be water-insoluble. The term "water-insoluble adhesive layer" within this disclosure may refer to an adhesive layer with a water-solubility of less than 0.25 g/dL, more specifically less than 0.1 g/dL and in particular less than 0.05 g/dL at 20 °C. In some embodiments, the applicator may not comprise a water-soluble slip layer and/or a water-absorbent layer. Further, in some embodiments, the carrier liner may be an outer layer of the applicator.

In some embodiments, the carrier liner adhesive layer may have a thickness between about 5 µm to about 200 µm, more specifically between about 20 µm to about 100 µm and in particular between about 30 µm to about 80 µm. The thickness may be measured from the carrier liner to the top of the carrier liner adhesive layer, excluding the pattern. The thickness of the carrier liner adhesive layer shall be measured at a part without pattern provided thereon.

In some embodiments, the applicator may comprise one or more compounds selected from the group of transcutol, an alkyl glycol or mixtures thereof, more specifically the one or more compounds may be selected from the group of 1,3-butylene glycol, pentylene glycol, hexylene glycol or mixtures. It has been found that 1,3-butylene glycol, pentylene glycol, hexylene glycol or mixtures thereof, in particular pentylene glycol, may result in a lower patchiness when used with a release liner comprising a film, compared to transcutol. Accordingly, in some embodiments, the one or more compounds may be pentylene glycol. The content of the one or more compounds selected from the group of transcutol, an alkyl glycol or mixtures thereof may be relatively low in the final applicator according to the second aspect. The applicator may undergo a heat treatment, wherein a significant amount of the one or more compounds may evaporate. The one or more compounds may furthermore evaporate during storage. In some embodiments, the pattern comprising the semi-permanent colorant may comprise between about 0.01 wt.-% to about 30% wt.-%, more specifically between about 0.05 wt.-% to about 25 wt.-%, and in particular between about 0.1 wt.-% to about 20 wt.-%, of the one or more compounds, relative to the total weight of the pattern. In some embodiments, the pattern comprising the semi-permanent colorant may comprise between about 0.01 wt.-% to about 5% wt.-%, more specifically between about 0.05 wt.-% to about 3 wt.-%, and in particular between about 0.1 wt.-% to about 2 wt.-%, of the one or more compounds, relative to the total weight of the pattern.

The pattern may comprise one or more additional colorants, in particular one or more temporary colorants. The term "temporary colorant" is well-known and i.a. attributed its common meaning in the art. Additionally or alternatively, the term "temporary colorant" may refer to a colorant that sits on top of the skin or, if it penetrates the skin, can diffuse out of the skin or can be washed off by, for example, water, soap, and/or isopropanol. As mentioned above, semi-permanent colorants such as genipin, may require time to (fully) develop their color. The additional colorant may visualize the applied temporary tattoo directly after application. In some embodiments, the one or more temporary colorants may be selected from the group consisting of: iron oxide black (Fe₃O4), iron oxide (FeO), carbon, logwood, ochre, cinnabar (HgS), cadmium red (CdSe), iron (III) oxide (Fe₂O₃), naphthol-AS pigment, disazodiarylide, disazopyrazolone, cadmium seleno-sulfide, cadmium yellow (CdS, CdZnS), curcuma yellow, chrome yellow (PbCrO₄), disazodiarylide, chromium oxide (Cr₂O₃), malachite [Cu₂(CO₃)(OH)₂], a ferrocyanide, a ferricyanide, lead chromate, monoazo pigment, Cu/Al phthalocyanine, Cu phthalocyanine, azure blue, cobalt blue, Cu-phthalocyanine, manganese violet (manganese ammonium pyrophosphate), an aluminum salt, quinacridone, dioxazine/carbazole, lead white (lead carbonate), titanium dioxide (TiO₂), barium sulfate (BaSO₄), zinc oxide, anthraquinone dyes and derivatives, annatto, caramel, β-carotene, bismuth citrate, disodium EDTA copper, potassium sodium copper chlorophyllin, dihydroxyacetone, bismuth oxychloride, guaiazulene, henna, ferric ammonium ferrocyanide, ferric ferrocyanide, chromium hydroxide green, chromium oxide greens, guanine, lead acetate, pyrophillite, mica, silver, titanium dioxide, aluminum powder, bronze powder, copper powder, ultramarines, manganese violet, zinc oxide, luminescent zinc sulfide, D&C Black Nos. 2 and 3, FD&C Blue No. 1 (e.g., acid blue 9), D&C Blue No. 4, D&C Brown No. 1, FD&C Green No. 3, D&C Green Nos. 5, 6, and 8, D&C Orange Nos. 4, 5, 10 and 11, FD&C Red Nos. 4, D&C Red Nos. 6, 7, 17, 21, 22, 27, 28, 30, 32, 33, 34, 36 and 40, D&C Violet No. 2, Ext. D&C Violet No.2, FD&C Yellow Nos. 5 and 6, D&C Yellow Nos. 7, 8, 10 and 11, and Ext. D&C Yellow No. 7. In some embodiments, the temporary colorant does not contain an amine group. In some embodiments, the temporary colorant is acid blue 9.

In some embodiments, the pattern may comprise a matrix component. The matrix component may provide mechanical stability to the pattern. Further, the semi-permanent colorant may be partially embedded in the matrix component, which may reduce the rate of semi-permanent colorant diffusion onto the second release liner. The matrix component may release the semi-permanent colorant when applied to the skin, e.g. by being slightly moistened by sweat. Further, the matrix component may increase the resolution of the printed image. Without wishing to be bound by theory, the matrix component may form hydrogen bonds with the semi-permanent colorant in the ink used for printing and/or during the drying process, which may reduce the mobility of the semi-permanent colorant and thereby blurring of the printed image, e.g. by preventing the semi-permanent colorant to diffuse on or into the carrier liner adhesive layer, in particular diffuse along the surface of the carrier liner adhesive layer.

In some embodiments, the matrix component may comprise a polyol. A polyol as used herein means an alcohol comprising at least three hydroxyl groups, in particular at least four hydroxyl groups. A polyol may include, for example, a mono-saccharide, di- saccharide, an oligosaccharide such as a tri-saccharide, sugar alcohol or other short chain polymeric polyol.

Optionally, a polyol as used herein may include polyol derivatives, which may have other hydrophilic groups incorporated therein, such as ethylene oxide derivatives of hydroxyl groups.

In some embodiments, the polyol (of the matrix component) may be a polyol capable of redispersing in aqueous media, in particular the polyol may be water-soluble. The term "water-soluble polyol" within this disclosure may refer to a polyol with a water-solubility of more than 0.5 g/dL, more specifically more than 10 g/dL and in particular more than 50 g/dL at 20°C. As mentioned above, the matrix component may release the semi-permanent colorant when moistened by sweat. Without wishing to be bound by theory, the matrix component when comprising a polyol may be partially solubilized when absorbing water, e.g. from sweat, allowing a higher rate of diffusion of the semi-permanent colorant towards the skin. Still, without wishing to be bound by theory, the matrix component may bind the semi-permanent colorant by forming hydrogen bonds with it, which are solubilized by water permeating into the pattern, in turn allowing for an increased movability of the semi-permanent colorant. The matrix component may also improve the transfer of the pattern to the skin, by acting as hygroscopic substance, in turn drawing sweat from the skin.

In some embodiments, the polyol may have a molar mass of at least 100 g/mol, more specifically at least 200 g/mol, and in particular at least 300 g/mol. The matrix component may be solid at 0°C, more preferably a solid at 20°C, still more specifically a solid at 30°C, in particular solid at 45°C. In some embodiments, the polyol may be solid at 0°C, more preferably a solid at 20°C, still more specifically a solid at 30°C, in particular solid at 45°C.

In some embodiments, the matrix component may be selected from the group consisting of: a sugar, a sugar alcohol, and a polymer. In some embodiments, the sugar may be selected from the group consisting of: trehalose, glucose, fructose, galactose, ribose, and xylose, trehalose, sucrose, lactotrehalose, galactotrehalose, 6-azidotrehalose, maltose, lactose, lactulose, cellobiose, chitobiose, a starch, glycogen, a cellulose, and a chitin. In some embodiments, matrix components may be trehalose. The one or more compounds mentioned above may also aid in solubilizing the semi-permanent colorant. When the solubilization of the semi-permanent colorant is improved, its distribution and embedding in the matrix component may be improved, which may also result in less patchiness, in particular after longer storage durations.

In some embodiments, the pattern comprising the semi-permanent colorant may comprise between about 1.5 wt.-% to about 30% wt.-%, more specifically between about 5 wt.-% to about 25 wt.-%, and in particular between about 10 wt.-% to about 20 wt.-%, of the matrix component, relative to the total weight of the pattern. In some embodiments, the pattern comprising the semi-permanent colorant may comprise between about 5 wt.-% to about 30% wt.-%, more specifically between about 10 wt.-% to about 25 wt.-%, and in particular between about 15 wt.-% to about 20 wt.-%, of the matrix component, relative to the total weight of the pattern. In some embodiments, the pattern comprising the semi-permanent colorant may comprise between about 30 wt.-% to about 90% wt.-%, more specifically between about 40 wt.- % to about 85 wt.-%, and in particular between about 50 wt.-% to about 75 wt.-%, of the semi-permanent colorant, relative to the total weight of the pattern.

In some embodiments, the pattern may further comprise one or more preservatives. In some embodiments, the one or more preservatives may be selected form the group consisting of: ascorbic acid, an ascorbate, a palmitate, citric acid, a benzoate, a benzoic acid, a propionate, propionic acid, a sorbate, sorbic acid, a salicylic acid, a salicylate, hexa-2,4-dienoic acid, a hexa-2,4-dienoate, formaldehyde, a formaldehyde releaser, formic acid and its salts, 3-acetyl-6-methylpyran-2,4-(3H)-dione and its salts, 3,3'-dibromo-4,4'-hexamethylenedioxydibenzamidine and its salts, thiomersal, phenylmercuric salts, undec-10-enoic acid and its salts, 1,3-bis (2-ethylhexyl) hexahydro-5-methyl-5-pyrimidine, 5-bromo-5-nitro-1,3-dioxane, bronopol, 2,4-dichlorobenzyl alcohol, 1-(4-chlorophenyl)-3-(3,4-dichlorophenyl) urea, chlorocresol, chloroxylenol, 5-chloro-2-(2,4- dichlorophenoxy) phenol, N,N"-methylenebis[N'-[3-(hydroxymethyl)-2, 5- dioxoimidazolidin-4-yl]urea], polyaminopropyl biguanide, methenamine, quaternium-15, climbazole, DMDM hydantoin, benzyl alcohol, 1-Hydroxy-4-methyl-6-(2,4,4-trimethylpentyl)-2-pyridon, piroctone olamine, bromochlorophene, o-cymen-5-ol, chlorophene, chloroacetaminde, methylchloroisothiazolinone, methylisothiazolinone, phenoxyisopropanol, chlorhexidine, chlorhexidine diacetate, chlorhexidine digluconate, chlorhexidine dihydrochloride, dimethyl oxazolidine, behentrimonium chloride, cetri-monium bromide, cetrimonium chloride, laurtrimonium bromide, laurtrimonium chloride, steartrimonium bromide, steartrimonium chloride, diazolidinyl urea, hexamidine, hexamidine diisethionate, hexamidine paraben, glutaral, 7-ethylbicyclooxazolidine, chlorphenesin, sodium hydroxymethylglycinate, silver chloride, benzethonium chloride, benzalkonium chloride, benza-lkonium bromide, benzalkonium saccharinate, benzylhemiformal, iodopropynyl butylcarbamate, biphenyl-2-ol and its salts, pyrithionine zinc, an erythorbate, a nitrite, ethylenediaminetetraacetic acid (EDTA), butylated hydroxyanisole (BHA), butylated hydroxytoluene (BHT), capryllic acid, dilauryl thiodipropionate, erythorbic acid, gum guaiac, methylparaben, a sulfite, a bisulfite, a metabisulfite, propyl gallatepy, propylparaben, stannous chloride, sulfur dioxide, thiodipropionic acid, an isothiazoline, a paraben, phenoxyethanol, ethylhexylglycerin, a glycols, and a tocopherol. In some embodiments, the one or more preservatives may be phenoxyethanol and ethylhexylglycerin. A 90:10 mixture of phenoxyethanol and ethylhexylglycerin is available under the brand name EUXYL^{®} PE9010, by the company Ashland Global Speciality Chemical Inc., USA.

In some embodiments, the applicator may have a shelf-life at ambient temperatures of at least 1 months, more specifically of at least 3 months and in particular of at least 6 months. It has been found that the applicators according to the present disclosure are particularly storage stable for a prolonged period of time.

### Manufacturing of the Applicator

In a second aspect, the present disclosure relates to a method for manufacturing an applicator for applying a pattern comprising a semi-permanent colorant. The method comprises applying a temporary tattoo ink on an adhesive layer provided on a carrier liner to provide a pattern. The carrier liner is in the shape of a trapezoid or an annular sector. A carrier liner with the shape of a trapezoid or an annular sector may result in a first section (such section being disclosed above) having the shape of a trapezoid or an annular sector.

In some embodiments according to the second aspect, the method optionally comprises cutting the carrier liner into a trapezoid or annular sector or providing the carrier liner as a trapezoid or annular sector. The cutting step may be necessary if the carrier liner is not provided already as a trapezoid or annular sector. In some embodiments according to the second aspect, the cutting step follows the application of the temporary tattoo ink and in particular may follow lamination of the carrier liner with its release liner.

In some embodiments, the method comprises providing a backing layer comprising an adhesive layer and laminating the carrier liner onto the adhesive layer of the backing layer. In some embodiments according to the second aspect, the backing layer may be provided with the release liner covering the part of the backing layer intended for the carrier liner to be laminated thereupon. The method may therefore comprise kiss-cutting the release liner of the backing layer in the shape of a trapezoid or annular sector and removing the kiss-cut release liner.

In some embodiments according to the second aspect, the method comprises cutting the backing layer to form a first section and a second section, wherein the first section has a trapezoidal or annular sector shape and the second section comprises at least one strap, wherein the first section is connected to the second section and the connection between the first section and the section is kiss-cut. In some embodiments according to the second aspect, cutting the backing layer includes forming a plurality of straps in the second section.

In some embodiments according to the second aspect, the method includes laminating the adhesive layer onto the backing layer, in particular wherein the adhesive layer is a double-sided transfer tape.

In some embodiments, the method comprises attaching a release liner to the adhesive layer, in particular by lamination, such that the release liner has a surface contacting the adhesive layer and the pattern.

In some embodiments according to the second aspect, applying the composition is performed by printing, in particular inkjet printing.

In some embodiments according to the second aspect, obtaining the applicator additionally may comprise a heat treatment, in particular following the application of the temporary tattoo ink on the adhesive layer and prior to attaching the release liner. The heat treatment may dry the ink, reducing the risk of the ink being smudged subsequently, in particular when the release liner is attached. Further, the heat treatment may improve adhesion between the pattern and the adhesive layer.

In some embodiments according to the second aspect, the heat treatment may be performed at a temperature between about 45°C to about 85°C, more specifically between about 55°C to about 75°C for a duration of between about 20 min to about 120 min, in particular between about 30 min to about 60 min.

In some embodiments according to the second aspect, the temporary tattoo ink comprises between about 30 wt.-% to about 90 wt.-% of one or more solvents, between about 1 wt.-% to about 30 wt.-% of one or more compounds selected from the group of transcutol, an alkyl glycol or mixtures thereof, and/or between about 1 wt.-% to about 20 wt.-% of one or more semi-permanent colorants.

In some embodiments according to the second aspect, the semi-permanent colorant may be present in an amount between about 1 wt.-% to about 20 wt.-%, more specifically between about 3 wt.-% to about 15 wt.-% and in particular between about 5 wt.-% to about 10 wt.-%, relative to the total weight of the temporary tattoo ink.

In some embodiments according to the second aspect, the one or more solvents may comprise one or more polar solvents, and in particular the solvents may be selected from the group of water, ethanol or mixtures thereof. The solvents may dissolve the semi-permanent colorant, e.g. genipin and/or other components, such as matrix components. The solvents, in particular their amounts, may also be adjusted to adjust the temporary tattoo ink's viscosity. In some embodiments according to the second aspect, the one or more solvents may be present in an amount between about 30 wt.-% to about 90% wt.-%, more specifically between about 50 wt.- % to about 85 wt.-%, and in particular between about 65 wt.-% to about 80 wt.-%, relative to the total weight of the temporary tattoo ink. In some embodiments according to the second aspect, the solvent may comprise water and ethanol, more specifically wherein the ratio between water and ethanol is between about 1:1 to about 1:2, more specifically between about 1:1.3 to about 1:1.8. The content of the one or more solvents may be relatively low in the final applicator according to the first aspect. The applicator may undergo a heat treatment (which is discussed below), wherein a significant amount of the solvents may evaporate (as mentioned above also for the one or more compounds). The solvents may also evaporate during storage. In some embodiments according to the first aspect, the pattern comprising the semi-permanent colorant may comprise between about 0.01 wt.-% to about 90% wt.-%, more specifically between about 0.05 wt.-% to about 85 wt.-%, and in particular between about 0.1 wt.-% to about 80 wt.-%, of the one or more solvents, relative to the total weight of the pattern. In some embodiments according to the first aspect, the pattern comprising the semi-permanent colorant may comprise between about 0.01 wt.-% to about 9% wt.-%, more specifically between about 0.05 wt.-% to about 5 wt.-%, and in particular between about 0.1 wt.-% to about 3 wt.-%, of the one or more solvents, relative to the total weight of the pattern.

The temporary tattoo ink may comprise one or more additional colorants, in particular one or more temporary colorants. In some embodiments according to the second aspect, the additional colorant, in particular the temporary colorant, may be present in an amount between about 0.05 wt.-% to about 1 wt.-%, more specifically between about 0.1 wt.-% to about 0.5 wt.-%, relative to the total weight of the temporary tattoo ink. The one or more additional colorants, in particular on or more temporary colorants, may be selected from those presented for the first aspect.

The temporary tattoo ink according to the second aspect may be used to print a pattern on an applicator. Accordingly, in some embodiments according to the second aspect, the temporary tattoo ink may be suitable for printing, in particular inkjet printing. In some embodiments according to the second aspect, the temporary tattoo ink may be an inkjet printer ink. The temporary tattoo ink may comprise additional ingredients which may improve its printability, e.g. by adjusting its viscosity and/or surface tension. In some embodiments according to the second aspect, the composition may be configured to have (or may have) a viscosity from about 1 to about 7 centipoise. In some embodiments according to the second aspect, the composition may be configured to have (or may have) a viscosity from about 3 to about 6 centipoise at 20 °C. In some embodiments according to the second aspect, the composition may be configured to have (or may have) a surface tension from about 20 to about 35 dyne/cm, more specifically between about 25 to about 30 dyne/cm at 20°C. In some embodiments according to the first aspect, the temporary tattoo ink may comprise less than 1 wt.-%, more specifically less than 0.1 wt.-% and in particular no or essentially no gelling agent, relative to the total weight of the temporary tattoo ink. In some embodiments according to the first aspect, the temporary tattoo ink may comprise less than 1 wt.-%, more specifically less than 0.1 wt.-% and in particular no or essentially no adhesive, relative to the total weight of the temporary tattoo ink. Gelling agents and adhesives may significantly increase the viscosity of the temporary tattoo ink making it unsuitable for printing, in particular inkjet printing. The viscosity may be measured for example using a Brookfield Ametek DVE Viscometer, model no. DVEELVTJ0 with a LTL adaptor for viscosities lower than 10 centipoise. The surface tension and contact angle may be measured using a Kruss Force Tensiometer using the du Noüy ring method according to ASTMD1331-20.

In some embodiments according to the second aspect, the temporary tattoo ink may comprise a surface active agent. The term "surface-active agent" is well known and i.a. attributed its common meaning in the art. Additionally or alternatively, the term "surface-active agent" may relate to a substance that tends to reduce the surface tension of a liquid in which it is dissolved. Non-limiting examples of a surface-active agent include an alkylbenzene sulfonate, an alkyl sulfate, an alkyl ether sulfate, a soap, an ethoxylate, an alkyl alcohol, a lignosulfonate, and a triglyceride. For example, a surface-active agent can include sodium dodecyl benzenesulfonate, lauryl sulfate, di-alkyl sulfosuccinate, dimethyl ether of tetradecyl phosphonic, lauryl monoethanol, glycerol diester (diglyceride), dodecyl betaine, abietic acid, polyethoxylated octyl phenol, 1,2-hexanediol (e.g., OPTIPHEN^{®} HD), and sorbitan monoester. In some embodiments according to the second aspect, the surface-active agent may be a diol and in particular 1,2-hexanediol. The surface-active agent, in particular the 1,2-hexandediol may reduce the surface tension of the temporary tattoo ink, which may improve the printing quality of the ink. Further, the surface-active agent, in particular the 1,2-hexandiol, may improve the skin penetration of the semi-permanent colorant. In some embodiments according to the second aspect, the surface active agent may be present in an amount between about 0.1 wt.-% to about 6% wt.-%, more specifically between about 0.5 wt.-% to about 4 wt.-%, and in particular between about 1 wt.-% to about 3 wt.-%, relative to the total weight of the temporary tattoo ink.

In some embodiments according to the second aspect, the temporary tattoo ink may comprise a matrix component, in particular one of the matrix component's presented for the first aspect. In some embodiments according to the second aspect, the matrix component may be present in an amount between about 0.1 wt.-% to about 6 % wt.-%, more specifically between about 0.5 wt.- % to about 4 wt.-%, and in particular between about 1 wt.-% to about 3 wt.-%, relative to the total weight of the temporary tattoo ink.

In some embodiments according to the second aspect, the temporary tattoo ink may further comprise one or more preservatives. In some embodiments according to the second aspect, the one or more preservatives may be present in an amount of about 0.5 wt.-% to about 1.5 wt.-%, relative to the total weight of the temporary tattoo ink. In some embodiments according to the second aspect, the one or more preservatives may be present in an amount of about 1 wt.-%, relative to the total weight of the temporary tattoo ink.

In some embodiments according to the first aspect, the temporary tattoo ink may comprise less than 1 wt.-%, more specifically less than 0.1 wt.-% and in particular no or essentially no gelling agent, relative to the total weight of the temporary tattoo ink. In some embodiments according to the first aspect, the temporary tattoo ink may comprise less than 1 wt.-%, more specifically less than 0.1 wt.-% and in particular no or essentially no adhesive, relative to the total weight of the temporary tattoo ink. Gelling agents and adhesives may significantly increase the viscosity of the temporary tattoo ink making it unsuitable for printing.

In a third aspect the present disclosure relates to a use of a backing layer for preventing or reducing wrinkling and/or buckling of an applicator comprising a pattern comprising a semi-permanent colorant during application of the applicator onto skin.

Although the present invention is defined in the attached claims, it should be understood that the present invention can also (alternatively) be defined in accordance with the following embodiments:
1. An applicator for applying a pattern comprising a semi-permanent colorant, wherein the applicator comprises:
   a carrier liner,
   and a pattern comprising a semi-permanent colorant,
   wherein the applicator comprises a first section, wherein the first section is in the shape of a trapezoid or an annular sector.
2. The applicator according to embodiment 1, wherein the applicator comprises a backing layer comprising a fabric.
3. The applicator according to any preceding embodiment, wherein the trapezoid or annular sector comprises at least one base comprising at least one notch.
4. The applicator according to embodiment 2 or 3, wherein the backing layer comprises a non-woven fabric, more specifically a non-woven thermoplastic and in particular a non-woven polyester.
5. The applicator according to any one of embodiments 2 to 4, wherein the backing layer, in particular the fabric, is pliant.
6. The applicator according to any one of embodiments 2 to 5, wherein the backing layer is configured to be adhesive to itself.
7. The applicator according to any one of embodiments 2 to 6, wherein the backing layer is configured to be non-adhesive to skin.
8. The applicator according to any one of embodiments 2 to 7, wherein the backing layer has a thickness between about 5 mils to about 30 mils.
9. The applicator according to any one of embodiments 2 to 8, wherein the backing layer has an elongation at yield of at least 110%, more specifically at least 115% and in particular at least 120%.
10. The applicator according to any one of embodiments 2 to 9, wherein the backing layer has an elongation at yield of between about 110% to about 200%, more specifically between about 115% to about 180 % and in particular between about 120% to about 160%, more specifically along an axis within the backing layer's planar extension and in particular along two perpendicular axis within the backing layer's planar extension..
11. The applicator according to any one of embodiments 2 to 10, wherein the backing layer has a tensile strength between about 0.5 MPa to about 10 MPa, more specifically between about 1 MPa to about 7 MPa and in particular between about 2 MPa to about 5 MPa.
12. The applicator according to any preceding embodiment, wherein the first section comprises at least two, and in particular two, notches in a first base and/or wherein the first section comprises at least one notch, more specifically at least two notches and in particular two notches in a second base.
13. The applicator according of embodiment 12, wherein the notches comprise an apex, wherein a line between an apex of a first notch on the first base and a second notch on the second base is a radius of the annular sector or trapezoid.
14. The applicator according to embodiment 13, wherein the first base has a first length between a first leg and a second leg of the trapezoid or annular sector, and wherein the distance between the first leg and the apex of the first notch is between about 0.2 times to about 0.4 times of the first length, more specifically wherein the first base comprises a first additional notch and wherein the distance between the second leg and the apex of the first additional notch is between about 0.2 times to about 0.4 times of the first length.
15. The applicator according to any one of embodiments 12 or 14, wherein the second base has a second length between the first leg and the second leg of the trapezoid or annular sector, and wherein the distance between the first leg and the apex of the second notch is between about 0.2 times to about 0.4 times of the second length, more specifically wherein the second base comprises a second additional notch and wherein the distance between the second leg and the apex of the second additional notch is between about 0.2 times to about 0.4 times of the second length.
16. The applicator according to any one of embodiments 3 to 15, wherein the backing layer comprises a fabric layer and an adhesive layer, wherein the adhesive layer is attached to the carrier liner in the first section.
17. The applicator according to embodiment 16, wherein the adhesive layer is attached to a first release liner in a second section.
18. The applicator according to any preceding embodiment, wherein the first section is connected to a second section comprising at least one strap, more specifically at least two straps and in particular three straps.
19. The applicator according to any preceding embodiment, wherein the second section, in particular the at least one strap, is attached to a leg of the trapezoid or of the annular sector.
20. The applicator according to any preceding embodiment, wherein a length of a leg of the trapezoid or of the annular sector is between about 5 cm to about 50 cm, more specifically between about 10 cm to about 40 cm.
21. The applicator according to any preceding embodiment, wherein the carrier liner is only present in the first section.
22. The applicator according to any preceding embodiment, wherein the carrier liner comprises a carrier liner adhesive layer, wherein the pattern is provided on and/or in the carrier liner adhesive layer.
23. The applicator according to embodiment 22, wherein the carrier liner adhesive layer is attached to a second release liner, in particular a different release liner as the release liner of the second section.
24. The applicator according to any preceding embodiment, wherein the carrier liner is non-porous.
25. A method for manufacturing an applicator for applying a pattern comprising a semi-permanent colorant, wherein the method comprises:
   applying a temporary tattoo ink on an adhesive layer provided on a carrier liner to provide a pattern, wherein the carrier liner is in the shape of a trapezoid or an annular sector.
26. The method according to embodiment 25, wherein method comprises cutting the carrier liner into a trapezoid or annular sector;
   or providing the carrier liner as a trapezoid or annular sector.
27. The method according to embodiment 25 or 26, wherein the method comprises providing a backing layer comprising an adhesive layer and laminating the carrier liner onto the adhesive layer of the backing layer.
28. The method according to any one of embodiments 25 to 27, wherein the cutting step follows the application of the temporary tattoo ink on the adhesive layer and in particular wherein the cutting step follows the lamination of the carrier liner with its release liner.
29. The method according to any one of embodiments 27 or 28, wherein the method comprises kiss-cutting a release liner of the backing layer in the shape of a trapezoid or annular sector and removing the kiss-cut release liner.
30. The method according to any one of embodiments 27 to 29, wherein the method comprises cutting the backing layer to form a first section and a second section, wherein the first section has a trapezoidal or annular sector shape and the second section comprises at least one strap, wherein the first section is connected to the second section and the connection between the first section and the section is kiss-cut.
31. The method according to one of embodiments 27 to 30, wherein cutting the backing layer includes forming a plurality of straps in the second section.
32. The method according to any one of embodiments 27 to 31, wherein the method includes laminating the adhesive layer onto the backing layer, in particular wherein the adhesive layer is a double-sided transfer tape.
33. The method according to any one of embodiments 25 to 32, wherein the method comprises attaching a release liner to the adhesive layer, in particular by lamination, such that the release liner has a surface contacting the adhesive layer and the pattern.
34. Use of a backing layer for preventing or reducing wrinkling and/or buckling of an applicator comprising a pattern comprising a semi-permanent colorant during application of the applicator onto skin.

## Claims

1. An applicator for applying a pattern comprising a semi-permanent colorant, wherein the applicator comprises:
a carrier liner,
and a pattern comprising a semi-permanent colorant,
wherein the applicator comprises a first section, wherein the first section is in the shape of a trapezoid or an annular sector.

2. The applicator according to claim 1, wherein the applicator comprises a backing layer comprising a fabric.

3. The applicator according to any preceding claim, wherein the trapezoid or annular sector comprises at least one base comprising at least one notch, in particular wherein the first section comprises at least two, and in particular two, notches in a first base and/or wherein the first section comprises at least one notch, more specifically at least two notches and in particular two notches, in a second base.

4. The applicator according to claim 2 or 3, wherein the backing layer comprises a non-woven fabric, more specifically a non-woven thermoplastic and in particular a non-woven polyester.

5. The applicator according to any one of claims 2 to 4, wherein the backing layer, in particular the fabric, is pliant.

6. The applicator according to any one of claims 2 to 5, wherein the backing layer has an elongation at yield of at least 110%, more specifically at least 115% and in particular at least 120%.

7. The applicator according to any one of claims 3 to 6, wherein the notches comprise an apex, wherein a line between an apex of a first notch on the first base and a second notch on the second base is a radius of the annular sector or trapezoid.

8. The applicator according to any one of claims 3 to 7, wherein the first base has a first length between a first leg and a second leg of the trapezoid or annular sector, and wherein the distance between the first leg and the apex of the first notch is between about 0.2 times to about 0.4 times of the first length, more specifically wherein the first base comprises a first additional notch and wherein the distance between the second leg and the apex of the first additional notch is between about 0.2 times to about 0.4 times of the first length.

9. The applicator according to any one of claims 3 to 8, wherein the second base has a second length between the first leg and the second leg of the trapezoid or annular sector, and wherein the distance between the first leg and the apex of the second notch is between about 0.2 times to about 0.4 times of the second length, more specifically wherein the second base comprises a second additional notch and wherein the distance between the second leg and the apex of the second additional notch is between about 0.2 times to about 0.4 times of the second length.

10. The applicator according to any one of claims 2 to 9, wherein the backing layer comprises a fabric layer and an adhesive layer, wherein the adhesive layer is attached to the carrier liner in the first section, in particular wherein the adhesive layer is attached to a first release liner in a second section.

11. The applicator according to any preceding claim, wherein the first section is connected to a second section comprising at least one strap, more specifically at least two straps and in particular three straps.

12. The applicator according to any preceding claim, wherein the carrier liner is only present in the first section.

13. The applicator according to any preceding claim, wherein the carrier liner is non-porous.

14. A method for manufacturing an applicator for applying a pattern comprising a semi-permanent colorant, wherein the method comprises:
applying a temporary tattoo ink on an adhesive layer provided on a carrier liner to provide a pattern, wherein the carrier liner is in the shape of a trapezoid or an annular sector.

15. Use of a backing layer for preventing or reducing wrinkling and/or buckling of an applicator comprising a pattern comprising a semi-permanent colorant during application of the applicator onto skin.
